# EUROPEAN PATENT SPECIFICATION

(11) **EP 2 977 439 B1**
(45) Date of publication and mention of the grant of the patent: **01.03.2017**
(21) Application number: 14178628.5
(22) Date of filing: 25.07.2014
(51) Int. Cl.: C12M 1/04, C12M 1/06, C12M 1/26, C12N 1/06

(54) **Method and apparatus for disruption of biomass cells**
Verfahren und Vorrichtung zur Spaltung von Biomassezellen
Procédé et appareil pour la désagrégation de cellules de biomasse

(43) Date of publication of application: 27.01.2016
(73) Proprietor: VITO NV (Vlaamse Instelling voor Technologisch Onderzoek NV), 2400 Mol (BE)
(72) Inventor: D'HONDT, Els, 2400 Mol (BE); ELST, Kathy, 2400 Mol (BE); GÜNERKEN, Emre, 63065 Offenbach (DE); GARCIA-GONZALEZ, Linsey, 2400 Mol (BE); WIJFFELS, René H, 6700AA Wageningen (NL); EPPINK, Michel H. M., 6700AA Wageningen (NL)
(74) Representative: Pronovem

(56) References cited:
- WO-A1-91/01367
- WO-A2-2004/108260
- US-A- 5 306 637
- US-A1- 2011 183 403

## Description

### Field of the Invention

The present invention relates to a method for disrupting suspended biomass material, a related apparatus (and stack of (parallel) systems) and the use thereof.

The present invention also relates to a method for solubilizing (valuable) cellular substances (from disrupted biomass material), a related apparatus (and stack of (parallel) systems) and the use thereof.

The present invention allows a subsequent selective extraction (or separation) of the obtained cellular substances (from the disrupted biomass material).

### Background of the Invention

To exploit a range of bioproducts from biological sources (including bacteria, yeast, algae, plant and animal tissues), liberation of intracellular products is of major importance where these products are not secreted into the suspending media by the microbial construct. The cell wall provides structural strength and is typically the key resistance to product liberation. The cell membrane, in turn, is readily disrupted in the absence of the cell wall.

In the art, a variety of disruption techniques are already available for the release of intracellular products.

Known mechanical cell disruption methods are, for example, ball milling, bead milling, high-pressure homogenizing, high-speed homogenizing, pressing of a sample at high pressure through a narrow aperture, or a method applied by means of an ultrasonic homogenizer.

A major drawback of these mechanical methods is the shear forces acting on the cells due to friction which, when trying to overcome them at high velocities, lead to a formation of heat and consequently generate a high temperature. In addition to high temperature, cavitational cell disruption methods (such as high speed homogenizing, high pressure homogenizing and ultrasonic homogenizer) cause extreme pressure differences. These may entail a harmful impact on the constituents of the evolving cellular extracts.

A more gentle method is for example provided by a physical decompression of cells, known in the art as (conventional) explosive decompression method. According to Henry's law, gas in cells is enriched at higher gas pressures and the cell membranes are thus caused to burst due to an abrupt pressure relief. This occurs because the dissolved gas cannot escape fast enough and bubbles out within the cells in form of growing gas bubbles. As a result, the mechanical load acting on the cell rises until the cell bursts and the cell content is released.

However, a drawback of the conventional explosive decompression method lies in that only cells that are relatively easy to break up can be efficiently disrupted which calls for additional application of non-mechanical disruption processes, like the use of enzymes, This in turn renders such a disruption process non-profitable for large-scale applications.

Mechanical methods have found greater commercial application compared with non-mechanical methods, because the latter infer operational and economic limitations at process scale. However, the disadvantages of the use of mechanical methods include a non-selective release of the product and micronization of the cell debris, complicating the further downstream process.

It is important to further integrate cell disruption with the formation and recovery (extraction) of the intracellular products.

Extraction of intracellular products is known in the art to be executed, for example, by means of supercritical fluids. This term relates to gases or liquids which are above their critical temperature and critical pressure that are defined in the relevant phase diagram of a pure substance. The benefit lies in an increased solubility for hardly soluble substances in the supercritical range. Moreover, solubility can still be controlled via alterations in pressure or temperature. However, the solubility of the supercritical form of a substance in water is generally lower than the solubility of the same substances in liquid form. Thus usually totally dried biomass is used in supercritical extraction methods known in the art.

An example is given by the decaffeination of a tea plant by means of supercritical CO₂, as described in WO 2008/05537 A1. Other applications in chemical industry and foodstuff industry have become well established methods as well, for example the extraction of oils, ginger, black pepper or chili powder by means of supercritical CO₂ or supercritical propane.

U.S. Patent No. 5,306,637 describes a method for cell disruption in which an enzymatic disruption is linked to a subsequent abrupt pressure relief causing the cells to burst and effectively releasing the valuable substance contained in them. U.S. Patent No. 5,306,637 uses supercritical or almost supercritical carbon dioxide, to which entraining agents are optionally added, for the disruption of microbial cells and for the extraction of the intracellular components, such as proteins or nucleic acids. However, here, the recovery of the used gas mixtures is extremely problematic and, therefore, this process is not applied on an industrial scale.

US 2011/0183403 describes a method for cell disruption of biogenic suspended raw materials by means of a combination of pressurization, atomization and decompression including a subsequent selective extraction and separation of cellular valuable substances. By this method, it is achieved that the cell disruption of the biogenic suspended raw material and the dissolution of the cellular valuable substances are performed simultaneously.

WO 2004/108260 describes an apparatus and a method for isolating a biologic product, such as plasmid DNA, from cells. The method involves lysing cells in a controlled manner separate insoluble components from a fluid lysate containing cellular components of interest, followed by membrane chromatographic techniques to purify the cellular components of interest. The process utilizes a unique lysis apparatus, ion exchange and, optionally, hydrophobic interaction chromatography membranes in cartridge form, and ultrafiltration. The process can be applied to any biologic product extracted from a cellular source. The process uses a lysis apparatus, including a high shear, low residence-time mixer for advantageously mixing a cell suspension with a lysis solution, a hold time that denatures impurities, and an air-sparging bubble mixer that gently yet thoroughly mixes lysed cells with a neutralization/precipiation buffer and floats compacted precipitated cellular material.

WO 91/01367 describes the supercritical or near-critical fluid disruption of microbial cells and extraction of Intracellular components and apparatuses associated therewith. First, a solvent that is a gas at ambient conditions and that has a critical temperature of between 0 and 100 °C is selected. This solvent is brought to near-critical pressure or higher and to near-critical temperature. The solvent then is combined with a slurry of cells to saturate the cells with the solvent under the prescribed conditions. Next, the pressure is released to cause a pressure drop which results in partial disruption of the cell membrane and release of solvent and other materials from the cell. Novel apparatus and associated methods are provided for carrying out the foregoing process continuously.

### Aims of the Invention

Aspects of the present invention envisage providing an improved method for disrupting suspended biomass material and a related apparatus (and stack of (parallel) systems), which overcome the disadvantages of prior art methods and apparatuses.

More particularly, it is envisaged to provide a method and a related apparatus (and stack of (parallel) systems) for a physical (non-mechanical), mild disruption of suspended biomass material.

Aspects of the present invention therefore envisage providing such method and related apparatus (and stack of (parallel) systems), which allow obtaining (or producing) (valuable) cellular substances from suspended biomass material, with a high throughput (compared to prior art methods and apparatuses).

Aspects of the present invention envisage providing such method for disrupting suspended biomass material and related apparatus (and stack of (parallel) systems), which allow for integration with a solubilization and/or subsequent selective extraction or separation of the obtained (valuable) cellular substances.

More particularly, it is envisaged to provide a subsequent solubilization of cellular substances from suspended biomass material and minimization of contaminating load by tuning the extraction ratio of the preferred product or biochemical. After solubilization, a subsequent selective extraction or separation of the obtained cellular substances is also envisaged.

Aspects of the present invention envisage providing such method (and related apparatus and stack of (parallel) systems) for disrupting suspended biomass material with (optionally) a solubilization and/or subsequent selective extraction or separation of the obtained cellular substances, which allow decreasing the total operational expenditure (OPEX) (or total cost) (compared to prior art methods and apparatuses) and being applicable on industrial scale.

### Summary of the Invention

According to aspects of the invention, there is therefore provided a method for disrupting suspended biomass material, as set out in the appended claims.

According to other aspects of the invention, there is provided an apparatus for disrupting suspended biomass material, as set out in the appended claims.

According to another aspect of the invention, there is provided a stack of (parallel) systems for disrupting suspended biomass material, based on the apparatus of the present invention.

According to other aspects of the invention, there is provided the use of the apparatus of the invention, as set out in the appended claims.

Advantageous aspects of the present invention are set out in the dependent claims.

### Short Description of the Drawings

Aspects of the invention will be described in more detail with reference to the appended drawings, wherein same reference numerals illustrate same features. In the drawings, not all alternatives and options are shown and therefore the invention is not limited to the content of the given drawings.
**Figure 1a** schematically represents an apparatus according to an aspect of the present invention, for disrupting suspended biomass material and, optionally, solubilizing the (valuable) cellular substances.
**Figure 1b** schematically represents an apparatus according to an aspect of the present invention, for disrupting suspended biomass material and, optionally, solubilizing the (valuable) cellular substances, said apparatus further comprising a pressure oscillation system (9).
**Figure 2** schematically represents a sparger system (7) used in the apparatus of **figures 1a** and **1b****.**
**Figure 3** depicts the comparison of the solubilization ratios (%) of different biochemical compositions, for untreated and treated suspensions of biomass using methods known in the art and according to the present invention.
**Figure 4** represents the energy consumption (in kWh/kg of dry biomass) versus the CO₂/suspension of biomass material ratio using the disruption method according to the invention.

### Description of the Invention

According to an aspect of the invention, there is provided an apparatus for disrupting suspended biomass material.

In the context of the present invention, disruption of (suspended, treated) biomass material refers to disruption of biomass cells. In the present description, this is also referred to as cell disruption. More particularly, during said disruption cell membranes as well as intracellular membranous structures are being disrupted. Intracellular membrane structures are, for example, thylakoid membranes or endoplasmic reticulum membranes.

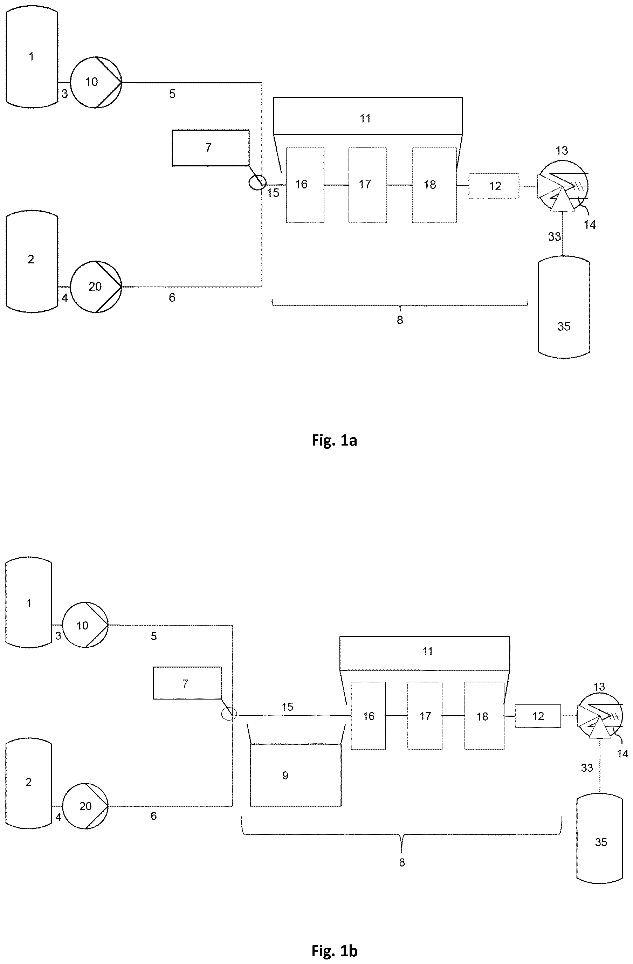

As illustrated schematically in **figure 1a****,** an apparatus according to the present invention comprises (or consists of) at least one first vessel (1) for holding (or providing) a (untreated) suspension of biomass material connected to at least one first high-pressure pump (10) by at least one tube (3), said first high-pressure pump (10) being adapted for pressurizing the suspension of biomass material coming from said first vessel (1); at least one second vessel (2) for holding (or providing) a pressurized gas disruption agent and/or pressurized liquid disruption agent connected to at least one second high-pressure pump (20) by at least one tube (4), said second high-pressure pump (20) being adapted for (further) pressurizing the gas and/or liquid disruption agent coming from said second vessel (2); wherein each of said first and second high-pressure pump (10,20) is connected by at least one tube (5,6) to at least one sparger system (7), said sparger system (7) being adapted for injecting a first stream (provided through said at least one tube (6)) of pressurized disruption agent into a second stream (provided through said at least one tube (5)) of a pressurized suspension of biomass material and for forming a micro-emulsion, wherein said sparger system (7) is further connected to a tubing system (8) for mixing said first and second streams, said tubing system (8) comprising a (at least one) primary tube (15) and (a) at least one hydrodynamic agitation system (11), and said tubing system (8) being further connected to a relief valve (13).

In the present description, a disruption (or disrupting) agent refers to a chemical compound used for disrupting biomass cells.

In the present invention, the gas and/or liquid disruption agent coming from said second vessel (2) is only present in liquid phase (or liquid state) after having passed second high-pressure pump (20), i.e. the pressure applied by second high-pressure pump (20) is such that no gas disruption agent is present in the system any more after having passed second high-pressure pump (20)). Said first stream of pressurized disruption agent is thus only present in liquid phase (or liquid state, i.e. being liquefied).

In the present invention, the disruption agents provided in second vessel (2) and the temperatures and pressures applied are such that no supercritical fluids can be present in the system until and/or after the relief valve (13). Optionally, in the invention, the temperature of the obtained micro- and/or nano-emulsion is only increased to the subcritical or critical point of the disruption agent, while transferring the process stream coming out of the relief valve (13) to a third vessel (35) at reduced or atmospheric pressure for (subsequently) solubilizing the (valuable) cellular substances (or cellular extracts) (from the disrupted biomass material).

Advantageously, the reduced pressure is comprised between (about) atmospheric pressure and the pressure (in the system) in between the hydrodynamic agitation system (11) and relief valve (13).

Advantageously, in an apparatus according to the present invention, said sparger system (7) is being adapted for injecting a first stream (provided through said at least one tube (6)) of pressurized disruption agent into a second stream (provided through said at least one tube (5)) of a pressurized suspension of biomass material and for forming a micro- and/or nano-emulsion.

In other words, an apparatus according to the invention as illustrated schematically in **figure 1a****,** comprises at least two high-pressure pumps (10,20), wherein at least one first pump (10) serves as high-pressure slurry pump and at least one second pump (20) serves as high pressure liquid/gas pump. Said pumps (10,20) each contain at least one inlet and at least one outlet. Said first pump (10) and second pump (20) are being configured (adapted) to pressurize a suspension of biomass material (or slurry) and a gas/liquid disruption agent, respectively, and to feed them further to the system (7) via tubes (5,6). Furthermore, the apparatus comprises at least one first vessel (1), serving as the feed source for an untreated suspension of biomass material, said at least one first vessel (1) having an outlet connected to the inlet of the high pressure slurry pump (10) via a tube (or conduit) (3). The apparatus comprises also at least one second vessel (2), serving as the feed source for pressurized gas/liquid disruption agent, said at least one second vessel (2) having an outlet connected to the inlet of high pressure liquid/gas pump (20) via a tube (or conduit) (4). The apparatus further comprises at least one sparger system (7) serving as the injector of pressurized (liquefied) disruption agent into a pressurized suspension of untreated biomass material (or adapted (configured) for injecting a pressurized (liquefied) disruption agent stream coming from high pressure liquid/gas pump (20) into the pressurized suspension of biomass material stream coming from high pressure slurry pump (10)) and being adapted for forming a micro- and/or nano-emulsion. The sparger system (7) is then sequentially connected by a primary tube (15) to at least one hydrodynamic agitation system (11) and a relief valve (13).

In the context of the present invention, the wording gas/liquid disruption agent refers to gas disruption agent (or disruption agent being in gas phase) and/or liquid disruption agent (or disruption agent being in liquid phase). In other words, depending on the temperature and/or the pressure in the system, the disruption agent in second vessel (2) is present in gas phase (or gas state) and/or in liquid phase (or liquid state).

In the context of the present invention, a stream of pressurized disruption agent coming out of the second high-pressure pump (20) (flowing through tube (6) to sparger system (7)) is only present in liquid phase (or liquid state, being liquefied) (i.e. the pressure applied by second high-pressure pump (20) is such that no gas disruption agent is present in the system any more after having passed second high-pressure pump (20)).

Advantageously, said first vessel (1) is connected to said first high-pressure pump (10) by a tube (3).

Advantageously, said second vessel (2) is connected to said second high-pressure pump (20) by a tube (4).

Advantageously, the at least one second vessel (2) is a tight-sealable vessel (i.e. being sealed off with respect to the surroundings).

According to an aspect of the present invention, an apparatus schematically illustrated in **figure 1a** can be used for disrupting suspended biomass material.

According to another aspect of the invention, there is provided a sparger system.

A sparger system of the invention is being adapted for injecting a first stream of pressurized (liquefied) disruption agent into a second stream of a pressurized suspension of biomass material so as to form a (mixed stream of) micro- and/or nano-emulsion (of disruption agent with biomass suspension).

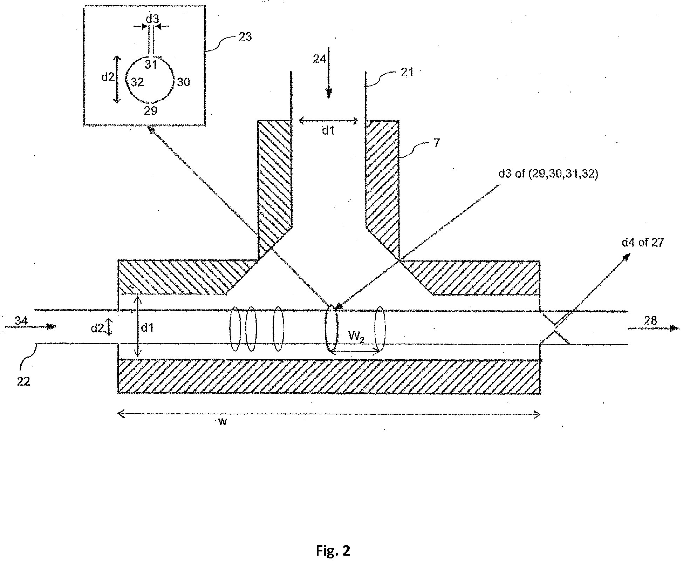

A sparger system (7) according to the present invention is schematically illustrated in **figure 2****.**

The sparger system (7) comprises a first sparger tube (21) having a diameter (d1) and having two inlets (24,34) and one outlet (28). Said first sparger tube (21) comprises a second sparger tube (22) placed along the direction formed by one of the inlets (34) and the outlet (28), said second sparger tube (22) having a diameter (d2) being two times smaller than the diameter (d1) of the first sparger tube. Said second sparger tube (22) comprises a distribution of sets (23) of first openings through the surface of the second sparger tube (22) along a width (w) (of first sparger tube (21)), said first openings having a diameter (d3) comprised between (about) 0.01 mm and (about) 1 mm, preferably between (about) 0.01 mm and (about) 0.2 mm, more preferably between (about) 0.025 mm and (about) 0.085 mm, even more preferably between (about) 0.05 mm and (about) 0.06 mm.

In the present description, said first sparger tube (21) can also be referred to as sparger shell (21).

Preferably, said first sparger tube (21) in sparger system (7) of the invention is T-shaped or Y-shaped, more preferably said first sparger tube (21) is T-shaped.

Advantageously, said second sparger tube (22) comprises one, two, three, or more sets (23) of first openings through the surface of the second sparger tube (22) along a width (w) (of sparger shell (21)).

More advantageously, the value of width (w) is comprised between the value of diameter (d3) of the first openings through the surface of the second sparger tube (22) and the total length of the second sparger tube (22) being inside (comprised within) first sparger tube (21). Even more advantageously, the value of width (w) is as small as possible to comprise all sets (23) of first openings (next to each other, along the width (w)), i.e. the value of width (w) satisfying the equation w=∑w₂.

More advantageously, said second sparger tube (22) comprises (only) one set (23) of first openings through the surface of the second sparger tube (22). In other words, (only) one set (23) of first openings is provided through the surface of the second sparger tube (22).

Without being bound by theory, providing more than two first openings (i.e. four, six, or more) per set of first openings, and/or providing more than one set of first openings (i.e. two, three, or more), will change the superficial velocity proportional to the parameter (1/the number of first openings) and the ratio of (first opening diameter (d3))²/(second sparger tube diameter (d2)²). Preferably, the parameter (1/the number of openings) is chosen smaller than the ratio of (first opening diameter (d3))²/(second sparger tube diameter (d2)²). In this preferable case, the superficial velocity of disruption agent will increase, which will lead to a pressure drop in the (second sparger tube (22) of) the sparger system (7). If the pressure drop due to the disruption agent passing through said first openings is higher than 50% of the pump pressure of the disruption agent (said pump pressure being provided by the second high-pressure pump (20) pressurizing the disruption agent coming from second vessel (2)), then, more advantageously, said second sparger tube (22) comprises more than one set (23) of first openings through the surface of the second sparger tube (22) (i.e. then, more advantageously, more than one set (23) of first openings is provided through the surface of the second sparger tube (22)).

Even more advantageously the value of width (w) equals (or is very close to) the value of diameter (d3) of the first openings through the surface of the second sparger tube (22).

Preferably, said second sparger tube (22) further comprises a second opening (27) at the outlet (28) of the second sparger tube (22), said second opening (27) having a diameter (d4) comprised between (about) 0.01 mm and (about) 0.6 mm, more preferably between (about) 0.1 mm and (about) 0.6 mm.

Advantageously, said first high-pressure pump (10) is connected by a tube (5) to the sparger system (7). More particularly, tube (5) is connected to inlet (34) of the sparger system (7).

Advantageously, said second high-pressure pump (20) is connected by a tube (6) to the sparger system (7). More particularly, tube (6) is connected to inlet (24) of the sparger system (7).

Preferably, diameter (d1) of first sparger tube (21) is comprised between (about) 1.6 mm (1/16 inch) and (about) 25.4 mm (1 inch), more preferably between (about) 3.2 mm (1/8 inch) and (about) 12.7 mm (1/2 inch), even more preferably diameter (d1) of first sparger tube (21) is (about) 6,4 mm (1/4 inch).

Preferably, diameter (d2) of second sparger tube (22) is comprised between (about) 0.8 mm (1/32 inch) and (about) 12.7 mm (1/2 inch), more preferably between (about) 1.6 mm (1/16 inch) and (about) 3.2 mm (1/8 inch), even more preferably diameter (d2) of second sparger tube (22) is (about) 3.2 mm (1/8 inch).

Preferably, diameter (d5) of tube (5) is comprised between (about) 0.8 mm (1/32 inch) and (about) 12.7 mm (1/2 inch), more preferably between (about) 1.6 mm (1/16 inch) and (about) 3.2 mm (1/8 inch).

Preferably, diameter (d6) of tube (6) is comprised between (about) 0.8 mm (1/32 inch) and (about) 12.7 mm (1/2 inch), more preferably between (about) 1.6 mm (1/16 inch) and (about) 3.2 mm (1/8 inch).

Advantageously, each set (23) of first openings in second sparger tube (22) comprises an even number (i.e. 2, 4, 6, or more) of first openings (or first orifices), said openings being located two by two opposite each other in one plane, said plane being perpendicular to the second sparger tube (22), each of the first openings being located through the surface of the second sparger tube (22).

More advantageously, each set (23) of first openings in second sparger tube (22) comprises at least two first openings (or first orifices) (29,31), said openings being located opposite each other in one plane, said plane being perpendicular to the second sparger tube (22), the first openings (29,31) being located through the surface of the second sparger tube (22) at a distance D1 satisfying the equation D1=(diameter (d2) of second sparger tube x n)/2 from the next opening of the set (or first openings (29,31) placed 180° from each other). Even more advantageously, each set (23) of first openings in second sparger tube (22) consists of two first openings (or first orifices) (29,31).

Even more advantageously, each set (23) of first openings in second sparger tube (22) comprises (or consists of) (only) two first openings (or first orifices) (29,31). In other words, (only) two first openings (29,31) are provided in each set (23) of first openings in second sparger tube (22).

More advantageously, each set (23) of first openings in second sparger tube (22) comprises at least four first openings (or first orifices) (29,30,31,32), said openings being located two by two opposite each other in one plane, said plane being perpendicular to the second sparger tube (22), each of the first openings (29,30,31,32) being located through the surface of the second sparger tube (22) at a distance D1 satisfying the equation D1=(diameter (d2) of second sparger tube x n)/4 from the next opening of the set (or first openings (29,30,31,32) placed 90° from each other). Even more advantageously, each set (23) of first openings in second sparger tube (22) consists of four first openings (or first orifices) (29,30,31,32).

Even more advantageously, each set (23) of first openings in second sparger tube (22) comprises (or consists of) (only) four first openings (or first orifices) (29,31). In other words, (only) four first openings (29,31) are provided in each set (23) of first openings in second sparger tube (22).

Advantageously, the first openings (29,30,31,32) are made by laser drilling.

In the present description, first openings (29,30,31,32) in second sparger tube (22) are also referred to as first orifices, (first) sparger holes, or (first) sparging holes.

In the present description, diameter (d3) of the first openings (29,30,31,32) in second sparger tube (22) is also referred to as the sparging hole diameter (of the sparging holes).

Advantageously, the sparger system (7) of the invention is a high-pressure sparger system.

In the present invention, the sparger system (7) serves as the injector of pressurized (liquefied) disruption agent into a pressurized suspension of biomass material. In other words, the sparger system (7) is adapted (configured) for injecting a first stream of pressurized (liquefied) disruption agent coming from high pressure liquid/gas pump (20) into a second stream of pressurized suspension of biomass material coming from high pressure slurry pump (10), thereby bringing (mixing) said first and second streams together (said streams being at high pressure) forming one (mixed) process stream.

In the context of the present invention, mixing refers to bringing two streams together, thereby bringing the two streams (only) in physical contact with each other. In other words, mixing is performed for (physically) "bringing together" two streams, without (chemical) reaction between said two streams, thereby forming one (mixed) process stream.

Advantageously, the first openings (29,30,31,32) in said second sparger tube (22) are adapted (configured) for producing micro (liquefied) disruption agent bubbles during said injection, thereby producing (forming) a micro-emulsion of mixed (liquefied) disruption agent with biomass suspension inside the sparger. Accordingly, using the sparger of the present invention, there is a relatively high efficiency of mass transfer between the (liquefied) disruption agent and the suspension to be mixed in the sparger.

In the present description, the stream of pressurized disruption agent coming out of the second high-pressure pump (20) (flowing further to the sparger system (6)) is referred to as 'liquefied disruption agent'. In other words, the pressure applied by second high-pressure pump (20) is such that no gas disruption agent is present in the system any more after the disruption agent having passed second high-pressure pump (20) (disruption agent is thus only present in liquid phase (or liquid state) after having passed second high-pressure pump (20)).

Advantageously, the sparger system (7) according to the invention is adapted for forming a micro-emulsion (in the sparger), said micro-emulsion comprising pressurized (liquefied) disruption agent and pressurized suspension of biomass material.

More advantageously, the first openings (29,30,31,32) in said second sparger tube (22) are adapted (configured) for producing micro and/or nano (liquefied) disruption agent bubbles during said injection, thereby producing (forming) a micro- and/or nano-emulsion of mixed (liquefied) disruption agent with biomass suspension inside the sparger. Accordingly, using the sparger of the present invention, there is a relatively high efficiency of mass transfer between the (liquefied) disruption agent and the suspension to be mixed in the sparger.

More advantageously, the sparger system (7) according to the invention is adapted for forming a micro- and/or nano-emulsion (in the sparger), said micro- and/or nano-emulsion comprising pressurized (liquefied) disruption agent and pressurized suspension of biomass material.

During the injection using the sparger of the invention, micro and/or nano (liquefied) disruption agent bubbles are passing through (or disruption agent is bubbling through) the pressurized suspension of biomass material flowing in the second sparger tube (22) (the disruption agent thereby getting in (physical) contact with the suspension of biomass material).

In the context of the present invention, micro bubbles are bubbles smaller than one millimeter in diameter, but larger than one micrometer.

In the context of the present invention, nano bubbles are bubbles smaller than one micrometer in diameter, but larger than one nanometer.

Advantageously, the second opening (27) in said second sparger tube (22) is adapted for further (or more effective) mixing micro and/or nano (liquefied) disruption agent bubbles with the pressurized suspension of biomass material (thereby further (or more effective) mixing the micro- and/or nano-emulsion formed in the sparger).

Advantageously, the second opening (27) in said second sparger tube (22) serves as outlet (28) for the process stream (through which the process stream exits the sparger system (7) and enters the tubing system (8)).

In the context of the present invention, process stream refers to the mixed stream (formed in the sparger) of disruption agent and suspension of biomass.

It will be convenient to note that the sparger according to the invention fundamentally differs from those already described in prior art. Indeed, during injection of a first stream of pressurized (liquefied) disruption agent into a second stream of pressurized suspension of biomass material using (a) sparger system(s) already described in the art, only droplets of disruption agent having a diameter comparable to the tube diameter are produced (e.g. having a diameter of (about) 3 mm to (about) 6 mm). Accordingly, there is a (very) low efficiency of mass transfer between the disruption agent and the suspension to be mixed in the sparger. In other words, no micro- and/or nano-emulsion of disruption agent mixed with suspension is formed when using a sparger known in the art. Using a sparger according to the present invention, to the contrary, relatively high efficiency of mass transfer is achieved (due to the formation of the micro and/or nano disruption agent bubbles), forming a micro- and/or nano-emulsion of disruption agent mixed with suspension of biomass, in the sparger.

Advantageously, the sparger system (7) of the invention is further connected to the primary tube (15) of tubing system (8) through the second sparger tube (22) having (or via) a second opening (second orifice) (27).

Otherwise stated, the sparger system (7) is connected to the primary tube (15) of the tubing system (8) via the second opening (27) at the outlet (28) of the second sparger tube (22). Second opening (27) is serving as outlet of the second sparger tube (22). The primary tube (15) is further sequentially connecting the hydrodynamic agitation system (11) and the relief valve (13).

Preferably, diameter (d7) of primary tube (15) is comprised between (about) 0.4 mm (1/64 inch) and (about) 12.7 mm (1/2 inch), more preferably between (about) 0.8 mm (1/32 inch) and (about) 12.7 mm (1/2 inch), even more preferably between (about) 1.6 mm (1/16 inch) and (about) 3.2 mm (1/8 inch), most preferably diameter (d7) of primary tube (15) is (about) 3.2 mm (1/8 inch).

Advantageously, the tubing system (8) is adapted for further mixing the process stream coming out the sparger system (7), thereby forming a (one) homogeneously mixed process stream (i.e. a homogenized mixture of disruption agent and suspension of biomass material, or a homogenized micro- and/or nano-emulsion of disruption agent with biomass suspension).

The apparatus of the present invention can be provided with or without including a pressure oscillation system (9).

More advantageously, the tubing system (8) further comprises a (or at least one) pressure oscillation system (9) before the (at least one) hydrodynamic agitation system (11). More particularly, the sparger system (7) is connected via primary tube (15) to a (or to at least one) pressure oscillation system (9) which is further connected (by said tube (15)) to the (at least one) hydrodynamic agitation system (11).

An apparatus of the invention further comprising a pressure oscillation system (9) is schematically illustrated in **figure 1b****.**

Preferably, the pressure oscillation system (9) comprises at least two (i.e. two, three, or more) orifices connected in series (by tube (15), primary tube (15) is thus comprised inside pressure oscillation system (9)).

More preferably, the pressure oscillation system (9) comprises (or consists of) three orifices connected in series (by tube (15), primary tube (15) is thus comprised inside pressure oscillation system (9)).

Preferably, each orifice in the pressure oscillation system (9) has a diameter (d8), said diameter (d8) being (about) four to (about) sixty-four times smaller, more preferably being (about) eight to (about) twenty-three times smaller, than the diameter (d7) of the primary tube (15).

Preferably, the diameter (d8) of the orifices in the pressure oscillation system (9) is comprised between (about) 0.02 and (about) 0.4 mm, more preferably between (about) 0.2 and (about) 0.4 mm.

Finding suitable diameters (d8) and (d9) of the orifice(s) and of the primary tube (15), respectively, in the pressure oscillation system (9), for use in the present invention depends on the characteristics of the disruption agent and of the suspension of biomass material used.

In the context of the present invention, an orifice of the pressure oscillation system (9) refers to a means for provoking Venturi effect. More particularly, such orifice is designed for controlling the characteristics of the stream flowing through it (especially to increase velocity and to reduce the pressure, due to the Bernoulli's equation) as it exits the orifice (and subsequently enters the next orifice or the primary tube). An orifice has a varying cross sectional area (compared to the primary tube), and is used to modify the flow of a fluid, for controlling the rate of flow, speed, and pressure of the stream that emerges from them.

In the present description, pressure oscillation system (9) can also be referred to as pressure and velocity oscillation system (or pressure/velocity system or pressure/velocity zone).

Advantageously, the pressure oscillation system (9) is adapted for further mixing the micro- and/or nano-emulsion formed in the sparger, thereby further increasing the efficiency of mass transfer between the (liquefied) disruption agent and the suspension already mixed in the sparger by further transferring the energy in between pressure-velocity-pressure oscillations, and enhancing the efficiency of the (resulting) cell disruption (i.e. the cell disruption achieved when the micro- and/or nano-emulsion has further passed the hydrodynamic agitation system (11) and the relief valve (13)).

Using the pressure oscillation system (9) is decreasing the cell number (i.e. more cells are being disrupted) and thus changing the final release ratio of cellular substances (i.e. optimizing solubilization of cellular substances), when compared with only using a hydrodynamic agitation system (11) for mixing the process stream.

More particularly, the extraction ratios of the cellular substances initially comprised in the biomass material can be tuned using the apparatus of the invention provided with or without a pressure oscillation system (9).

In the present invention, a standard hydrodynamic agitation system (11) known in the art is used.

Advantageously, the hydrodynamic agitation system (11) comprises at least one (i.e. one, two, three, or more) subsidiary tubing system (16) connected by the (at least one) primary tube (15), wherein the diameter (d9) of the tubes of the subsidiary tubing system (16) is one to two times smaller, more advantageously two times smaller, than the diameter (d7) of the primary tube (15) (in case the process flow splits in two).

More advantageously, the hydrodynamic agitation system (11) comprises (or consists of) three subsidiary tubing systems (16,17,18) connected by the (at least one) primary tube (15), wherein the diameter (d9) of the tubes of the subsidiary tubing systems (16,17,18) is one to two times smaller, even more advantageously two times smaller, than the diameter (d7) of the primary tube (15) (in case the process flow splits in two).

Preferably, the diameter (d9) of the tubes of the subsidiary tubing system(s) (16,17,18) is comprised between (about) 0.4 mm (1/64 inch) and (about) 12.7 mm (1/2 inch), more preferably between (about) 0.4 mm (1/64 inch) and (about) 6,4 mm (1/4 inch), even more preferably diameter (d9) of the tubes of the subsidiary tubing system(s) (16,17,18) is (about) 1.6 mm (1/16 inch) (in case the process flow splits in two).

Advantageously, the hydrodynamic agitation system (11) is adapted for further mixing (or hydrodynamic mixing) the process stream, thereby increasing the mass transfer of pressurized disruption agent to the suspension of biomass material and producing a more homogeneous micro- and/or nano-emulsion.

In the context of the present invention, a relief valve (13) refers to a means or valve to control or limit the pressure in a system or in a vessel.

In the apparatus of the present invention, the relief valve (13) is adapted for (suddenly) decreasing the pressure in the system, i.e. to decrease the pressure of the process stream passing through and coming out of the relief valve (13) to reduced pressure or to atmospheric pressure.

Using the apparatus of the invention, and passing a micro- and/or nano-emulsion of (liquefied) disruption agent with biomass suspension formed in the sparger system (7) sequentially through a hydrodynamic agitation system (11) and a relief valve (13), thereby exposing the biomass material to an explosive (or rapid) decompression (i.e. suddenly drop in pressure), results in disrupting the treated biomass material.

Optionally, using the apparatus of the invention, the micro- and/or nano-emulsion of disruption agent with biomass suspension formed in the sparger system (7) is first passed through a pressure oscillation system (9) before sequentially passing through a hydrodynamic agitation system (11) and a relief valve (13). Thereby the biomass material is exposed to an explosive (or rapid) decompression, resulting in disruption of the treated biomass material.

Advantageously, in the apparatus of the present invention, the tubing system (8) (after the sparger system (7)) further comprises at least one means (12) for measuring pressure.

Preferably, said means (12) for measuring pressure is comprised (or present) in between the (at least one) hydrodynamic agitation system (11) and the relief valve (13) (and connected to them via primary tube (15)).

Advantageously, the at least one means (12) for measuring (and monitoring) pressure is a pressure gauge.

Advantageously, in the apparatus of the present invention, the first and second high-pressure pumps (10,20) comprise a pressure gauge.

Advantageously, in the apparatus of the present invention, the relief valve (13) comprises a means (14) for heating a process stream coming out of the tubing system (8).

Advantageously, said relief valve (13) is further connected to a third vessel (35) by a (outflow) tube (33).

Advantageously, said third vessel (35) is used for collecting the process stream coming out of the relief valve (13).

In the context of the present invention, a means for heating (14) refers to a heating system being adapted for heating or increasing the temperature of a (homogeneously mixed) process stream (or output stream) coming out of the tubing system (8) (said heating system being able to resist to very high pressures).

Advantageously, the means (14) for heating comprised in relief valve (13) is adapted for increasing the temperature of the micro- and/or nano-emulsion (process stream coming out of the tubing system (8)) to avoid a (possible) freezing effect of rapid decompression (or to avoid freezing effect of pressure decrease, to avoid ice formation, due to using relief valve (13)).

Alternatively, the means (14) for heating comprised in relief valve (13) is adapted for increasing the temperature of the micro- and/or nano-emulsion (process stream coming out of the tubing system (8)) to the subcritical or critical point (or even higher temperature) of the disruption agent. Increasing the temperature of the micro- and/or nano-emulsion to the subcritical or critical point of the disruption agent, while transferring the process stream coming out of the relief valve (13) to a third vessel (35) (via tube (33)) at reduced or atmospheric pressure solubilizes (valuable) cellular substances (or cellular extracts) (from the disrupted biomass material).

According to the present invention, an apparatus is thus provided for disrupting suspended biomass material and (optionally) solubilizing the (valuable) cellular substances (from the disrupted biomass material).

Preferably, the diameter (d10) of outflow tube (33) is comprised between (about) 0.1 mm and (about) 12.7 mm, more preferably between (about) 0.1 mm and (about) 3.175 mm.

Advantageously, the material used for the apparatus of the invention is cold manufactured stainless steel (or 304 SS, 304L SS, 316 SS, 316L SS)

According to an aspect of the present invention, an apparatus schematically illustrated in **figure 1a and 1b** can be used for disrupting suspended biomass material.

Advantageously, an apparatus schematically illustrated in **figure 1a and 1b** can be used for disrupting suspended biomass material and (optionally) solubilizing the (obtained, valuable) cellular substances.

In the present invention, the (solubilized) cellular valuable substances (from the disrupted biomass material) can be further (partially) isolated (or (partially) separated) and selective extracted.

Advantageously, (solubilized) proteins can be further (partially) isolated (or (partially) separated) and selective extracted from disrupted biomass material obtained via a mild disruption method of the invention.

According to an aspect of the invention, there is provided a stack (or arrangement) of (parallel) systems (devices, apparatuses) for disrupting suspended biomass material.

Advantageously, a stack (or arrangement) of (parallel) systems (devices, apparatuses) can be used for disrupting suspended biomass material and (optionally) solubilizing the (obtained, valuable) cellular substances.

Advantageously, said stack (or arrangement) of (parallel) systems is based on the apparatus of the present invention being discussed in detail above.

A stack (or arrangement) of (parallel) systems according to the present invention comprises a (one) first vessel for holding (or providing) a (untreated) suspension of biomass material connected to a (one) first high-pressure pump, said first high-pressure pump being adapted for pressurizing the suspension of biomass material coming from said first vessel; a (one) second vessel for holding (or providing) a pressurized gas disruption agent and/or pressurized liquid disruption agent connected to a (one) second high-pressure pump, said second high-pressure pump being adapted for (further) pressurizing the gas and/or liquid disruption agent coming from said second vessel; wherein the first and second high-pressure pumps are connected to a series of (two, three, or more) (parallel) systems via (using) a distribution manifold, each of said parallel systems comprising at least one sparger system of the present invention, said sparger system being adapted for injecting a first stream of pressurized disruption agent into a second stream of a pressurized suspension of biomass material and for forming a micro- (and/or nano-)emulsion, wherein said sparger system is further connected to a tubing system for mixing said first and second streams, said tubing system comprising a (at least one) primary tube and (a) at least one hydrodynamic agitation system, and said tubing system being further connected to a relief valve.

Each of the (two, three, or more) (parallel) systems can be provided with or without including a pressure oscillation system.

More advantageously, the tubing system in each of the (two, three, or more) (parallel) systems further comprises a (or at least one) pressure oscillation system before the (at least one) hydrodynamic agitation system.

Advantageously, the relief valve of each of the (two, three, or more) (parallel) systems is connected to a (one) third vessel via (using) a distribution manifold.

Advantageously, said third vessel is used for collecting the process streams coming out of each of the relief valves (of each of the (two, three, or more) (parallel) systems).

The stack (or arrangement) of (parallel) systems of the present invention can be used on industrial scale.

According to an aspect of the invention, there is provided a method for disrupting suspended biomass material.

A method according to the invention for disrupting suspended biomass material comprises the steps of:
- providing a suspension of biomass material in a first vessel (1), said suspension having a dry cell weight being comprised between 0.1% and 15% (w/w);
- pressurizing said suspension to a pressure (p1) being comprised between 7500 kPa and 450000 kPa by using a first high-pressure pump (10);
- providing a pressurized gas disruption agent and/or a pressurized liquid disruption agent in a second vessel (2) (the gas disruption agent and/or liquid disruption agent in the second vessel (2) being at a pressure (p0) comprised between (about) 5000 kPa (50 bar) and (about) 6000 kPa (60 bar));
- pressurizing said gas and/or liquid disruption agent to a pressure (p2) being comprised between 7500 kPa and 450000 kPa by using a second high-pressure pump (20), said pressure (p2) being higher than said pressure (p1);
- injecting a stream of said pressurized disruption agent into a stream of said pressurized suspension of biomass material by using a sparger system (7), thereby bringing said two streams together and forming a micro-emulsion;
- passing said micro-emulsion sequentially through a hydrodynamic agitation system (11) and a relief valve (13).

In a method of the invention, a (untreated) suspension of biomass material is provided in a first vessel (1), said suspension having a dry cell weight being comprised between (about) 0.1% and (about) 15% (w/w). A stream of said suspension is then flowing through tube (3) to a first high-pressure pump (10). Said suspension is then pressurized to a pressure (p1) being comprised between (about) 7500 kPa (75 bar) and (about) 450000 kPa (4500 bar), by using the first high-pressure pump (10). Furthermore, a pressurized gas and/or pressurized liquid disruption agent is provided in a second vessel (2). The gas disruption agent and/or liquid disruption agent in the second vessel (2) is present in said vessel (2) at a pressure (p0) being comprised between (about) 5000 kPa (50 bar) and (about) 6000 kPa (60 bar). A stream of said pressurized gas disruption agent and/or pressurized liquid disruption agent is then flowing through tube (4) to a second high-pressure pump (20). Said gas and/or liquid disruption agent (coming from second vessel (2)) is then (further) pressurized to a pressure (p2) being comprised between (about) 7500 kPa (75 bar) and (about) 450000 kPa (4500 bar), by using the second high-pressure pump (20). In a method of the invention, said pressure (p2) is higher than said pressure (p1). A stream of said pressurized suspension of biomass material coming out of the first high-pressure pump (10) is then flowing through tube (5). A stream of said pressurized (liquefied) disruption agent coming out of the second high-pressure pump (20) is flowing through tube (6). The stream of said pressurized (liquefied) disruption agent is then injected (or sparged) into the stream of said pressurized suspension of biomass material by using a sparger system (7) according to the invention, thereby bringing said two streams together and forming a micro-emulsion (in the sparger system (7)). Said (formed) micro-emulsion is then sequentially passed through a hydrodynamic agitation system (11) and a relief valve (13).

In the present invention, the gas and/or liquid disruption agent coming from said second vessel (2) is only present in liquid phase (or liquid state) after having passed second high-pressure pump (20), said stream of pressurized disruption agent flowing after (or coming out of) second high-pressure pump (20) is thus only present in liquid phase (or liquid state, i.e. being liquefied).

Advantageously, in a method of the invention, a micro- and/or nano-emulsion is formed in the sparger system (7). Said (formed) micro- and/or nano-emulsion is then sequentially passed through a hydrodynamic agitation system (11) and a relief valve (13).

Preferably, the dry cell weight of the (untreated) suspension of biomass is comprised between (about) 0.1% and (about) 6% (w/w), more preferably between (about) 0.5 % and (about) 4 % (w/w), even more preferably between (about) 1.5 % and (about) 2.5 % (w/w).

Preferably, said pressure (p1) is comprised between (about) 7500 kPa and (about) 25000 kPa, more preferably between (about) 20000 kPa and (about) 25000 kPa.

Preferably, said pressure (p2) is comprised between (about) 7500 kPa and (about) 25000 kPa, more preferably between (about) 20000 kPa and (about) 25000 kPa.

According to the invention, said pressure (p2) is higher than said pressure (p1).

In a method of the present invention, the disruption agent in second vessel (2) is present in gas phase (or gas state) and/or in liquid phase (or liquid state), depending on the temperature and/or the pressure in the system.

In a method of the present invention, a stream of pressurized disruption agent coming out of the second high-pressure pump (20) (flowing through tube (6) to sparger system (7)) is only present in liquid phase (or liquid state) (i.e. the pressure applied by second high-pressure pump (20) is such that no gas disruption agent is present in the system any more after having passed second high-pressure pump (20)).

Preferably, the superficial velocity of the mixed stream (or process stream) of disruption agent and suspension of the biomass material inside the tube (15) is lower than (about) 0.51 m/s, more preferably lower than (about) 0.1 m/s, even more preferably lower than (about) 0.05 m/s, and most preferably lower than (about) 0.03 m/s.

It is well within the practice of those skilled in the art that sample flow rates can be changed by changing the pressure in the system.

With the method of the present invention, a same yield of cell disruption can be achieved using higher pressures combined with lower flow rates, or using lower pressures combined with higher flow rates.

It is to be noted that a method of the invention is provided obtaining (valuable) cellular substances from suspended biomass material with a high throughput (and low contact time). It is believed that the high throughput (and low contact time) is due to the superficial velocities of disruption agent and suspension inside the tubes (5,6) of the system, the diameter (d7) of primary tube (15), and the diameter (d9) of the tubes of the subsidiary tubing system(s) (16,17,18) (in the hydrodynamic agitation system (11)).

Biomass material can be used to produce high-value bioactive molecules.

Advantageously, in a method of the invention, biomass material comprises (or consists of) biogenic source material.

More advantageously, said biomass material comprises (or consists of) single cell (or unicellular) biomass material.

Advantageously, the biomass material is suspended in a water-based environment (i.e. being in an aqueous suspension).

Advantageously, the suspension of biomass material comprises (or consists of) microalgae, bacteria, yeast/fungi cell, plant cell, animal cell, insect cell, or cyanobacteria.

Advantageously, the size of the particulates of the biomass material (cells) (in the suspension) is larger than (about) 1 µm.

Preferably, the ratio of the disruption agent to the suspension of biomass material is comprised between (about) 0.001 and (about) 0.95 kg/kg, more preferably between (about) 0.005 and (about) 0.95 kg/kg, even more preferably between (about) 0.18 kg/kg and (about) 0.5 kg/kg, and most preferably between (about) 0.23 kg/kg and (about) 0.45 kg/kg.

In the context of the present invention, the ratio of disruption agent to suspension (of biomass material) refers to the ratio of disruption agent mass flow rate to (biomass material) suspension mass flow rate.

It is to be noted that in a method of the invention the total amount of disruption agent used is reduced, when compared to prior art methods. Consequently the method of the invention consumes less energy, thereby reducing the total operational expenditure (or total cost), when compared to prior art methods.

Advantageously, the sparger system (7) according to the invention is adapted for forming a micro-emulsion (in the sparger), said micro-emulsion comprising pressurized disruption agent and pressurized suspension of biomass material.

More advantageously, the sparger system (7) according to the invention is adapted for forming a micro- and/or nano-emulsion (in the sparger), said micro- and/or nano-emulsion comprising pressurized disruption agent and pressurized suspension of biomass material.

According to a method of the invention and referring to **figure 2****,** the stream of the pressurized (liquefied) disruption agent flowing in tube (6) is injected (or sparged) into the stream of the pressurized suspension of biomass material flowing in tube (5) by using a sparger system (7), thereby bringing said two streams together. Tube (5) is connected to inlet (34) of the sparger system (7). Tube (6) is connected to inlet (24) of the sparger system (7).

More particularly, the stream of the pressurized (liquefied) disruption agent flowing in tube (6) is injected via inlet (24) of the sparger system (7) into the stream of the pressurized suspension of biomass material flowing through inlet (34) in the second sparger tube (22). The injection of the disruption agent into the suspension is performed through openings (29,30,31,32) through the surface of the second sparger tube (22).

Advantageously, in a method of the invention, the stream of said pressurized disruption agent is injected into the stream of said pressurized suspension through openings (29,30,31,32) (through the surface) of ((second) sparger tube (22) having) a diameter (d3) comprised between (about) 0.01 mm and (about) 1 mm.

Preferably, the diameter (d3) is comprised between (about) 0.01 mm and (about) 0.2 mm, more preferably between (about) 0.025 mm and (about) 0.085 mm, even more preferably between (about) 0.05 mm and (about) 0.06 mm.

Advantageously, the sparger system (7) of the invention is a high-pressure sparger system (a sparger system for high pressure processes). The pressure inside sparger system (7) is pressure (p2), set by second high-pressure pump (20) and further depends on the disruption agent flow rate in tube (6) and the number and diameter of the (first) sparger holes.

Advantageously, in a method of the invention, after the step of using the sparger system (7) and before the step of passing sequentially through the hydrodynamic agitation system (11) and the relief valve (13), said micro-emulsion is passing through a pressure oscillation system (9).

More advantageously, in a method of the invention, after the step of using the sparger system (7) and before the step of passing sequentially through the hydrodynamic agitation system (11) and the relief valve (13), said micro- and/or nano-emulsion is passing through a pressure oscillation system (9).

Advantageously, said micro- and/or nano-emulsion is then passing through the sparger system (7) to the primary tube (15) of a pressure oscillation system (9) which is further connected (by said tube (15)) to the (at least one) hydrodynamic agitation system (11). More particularly, said micro- and/or nano-emulsion is passing through a second opening (27) of its second sparger tube (22) having diameter (d4) to the primary tube (15) of a pressure oscillation system (9) which is further connected to a hydrodynamic agitation system (11).

Preferably, said second opening (27) has a diameter (d4) comprised between (about) 0.01 mm and (about) 0.6 mm, more preferably between (about) 0.1 mm and (about) 0.6 mm.

Advantageously, the pressure oscillation system (9) is adapted for further mixing the micro- and/or nano-emulsion formed in the sparger (thereby further increasing the efficiency of mass transfer between the (liquefied) disruption agent and the suspension already mixed in the sparger by further transferring the energy in between pressure-velocity-pressure oscillations and enhancing the efficiency of the (resulting) cell disruption). In other words, in the pressure oscillation system (9), the biomass material (comprised in the micro- and/or nano-emulsion passing through it) is prepared for actually being disrupted when subsequentially passing through the hydrodynamic agitation system (11) and exiting from the relief valve (13). Using the pressure oscillation system (9) is decreasing the cell number (i.e. more cells are being disrupted) and thus changing the final release ratio of cellular substances (i.e. optimizing solubilization of cellular substances), when compared with only using a hydrodynamic agitation system (11) for mixing the process stream.

Preferably, in the pressure oscillation system (9) the micro- and/or nano-emulsion passes through at least two orifice(s) (being connected in series by tube (15)), each orifice having a diameter (d8) being (about) four to (about) sixty-four times smaller, more preferably being (about) eight to (about) twenty-three times smaller, than the diameter (d7) of its primary tube (15).

Advantageously, the hydrodynamic agitation system (11) is adapted for further mixing (or hydrodynamic mixing) the process stream (thereby increasing the mass transfer of pressurized disruption agent to the suspension of biomass material and producing a more homogeneous micro- and/or nano-emulsion).

Advantageously, a method of the present invention further comprises a step of passing the micro- and/or nano-emulsion formed in sparger system (7) through a means (12) for measuring pressure.

Advantageously, the micro- and/or nano-emulsion passes said means (12) for measuring pressure in between the hydrodynamic agitation system (11) and the relief valve (13).

More particularly, in a method of the present invention, the micro- and/or nano-emulsion formed in sparger system (7) is further passing through a means (12) for measuring pressure after coming out of the hydrodynamic agitation system (11) and before passing through the relief valve (13).

In a method of the present invention, the relief valve (13) (suddenly) decreases the pressure in the system, i.e. decreases the pressure of the process stream passing through and coming out of the relief valve (13) to reduced pressure or to atmospheric pressure.

In the context of the present invention, reduced pressure refers to a pressure being higher than atmospheric pressure and being lower than the pressure measured by the means (12) for measuring pressure (or by pressure gauge (12)) in between the hydrodynamic agitation system (11) and relief valve (13).

Passing a micro- and/or nano-emulsion of disruption agent with biomass suspension formed in the sparger system (7) sequentially through a hydrodynamic agitation system (11) and a relief valve (13), thereby exposing the biomass material to an explosive (or rapid) decompression (i.e. suddenly drop in pressure), results in disrupting the treated biomass material.

Advantageously, the relief valve (13) comprises a means (14) for heating a process stream coming out of the tubing system (8) and passing through the relief valve (13).

In other words, after having formed a micro- and/or nano-emulsion in the sparger system (7) of the invention, and sequentially passing said micro- and/or nano-emulsion through a hydrodynamic agitation system (11) and a relief valve (13), the biomass material (comprised in the micro- and/or nano-emulsion) is being disrupted.

Optionally, the micro- and/or nano-emulsion of disruption agent with biomass suspension formed in the sparger system (7) is first passed through a pressure oscillation system (9) before sequentially passing through a hydrodynamic agitation system (11) and a relief valve (13). Thereby the biomass material is exposed to an explosive (or rapid) decompression, resulting in disruption of the treated biomass material.

More particularly, a method is provided for disrupting suspended biomass material.

By passing a micro- and/or nano-emulsion (formed in sparger system (7)) sequentially through a hydrodynamic agitation system (11) and a relief valve (13), and optionally first passing through a pressure oscillation system (9) before sequentially passing through a hydrodynamic agitation system (11) and a relief valve (13), in a method of the invention, no high shear forces (or stress) act on the biomass material to be disrupted. Therefore, by performing a method of the invention, a non-mechanical, physical, mild (i.e. more gentle) disruption of biomass material is provided (when compared to cell disruption methods described in the art).

Preferably, in a method of the invention, the suspension of biomass material is saturated by the pressurized disruption agent by hydrodynamic mixing.

Alternatively, the suspension of biomass material is oversaturated by the pressurized disruption agent by hydrodynamic mixing.

Alternatively, the suspension of biomass material is not saturated by the pressurized disruption agent by hydrodynamic mixing.

In the context of the present invention, pretreatment may (further) reduce the energy requirement for performing a method of the invention, and may avoid micronization of cell debris, and influence cell deactivation.

Preferably, in a method of the invention, the suspension of biomass material is pretreated by centrifugation, scrubbing, washing, filtering, crushing, grinding, ball mill, bead mill, high pressure homogenizer, high speed homogenizer, ultrasonic homogenizer, microwave, ultrasonic bath, french press, pulsed electric fields, pulsed arc, microfluidizer, or heat only treatment, prior to be provided in the first vessel (1).

More preferably, the suspension of biomass material is pretreated by scrubbing, bead mill, high pressure homogenizer, high speed homogenizer, or microwave, prior to be provided in the first vessel (1).

Alternatively, in a method of the invention, the suspension of biomass material is not pretreated.

Advantageously, in a method of the invention, the temperature of the micro- and/or nano-emulsion passing the relief valve (13) is increased (by the means (14) for heating comprised in said relief valve (13)) to avoid a (possible) freezing effect of rapid decompression (or to avoid freezing effect of pressure decrease, to avoid ice formation, due to using relief valve (13)).

Alternatively, in a method of the invention, simultaneously with (i.e. during, together with) the step of passing the micro- and/or nano-emulsion through the relief valve (13), the temperature of the micro- and/or nano-emulsion is increased to the subcritical or critical point (or even higher temperature) of the disruption agent and the process stream coming out of the relief valve (13) is transferred to a third vessel (35) (via tube (33)) at reduced or atmospheric pressure (using relief valve (13)) (thereby solubilizing (valuable) cellular substances (or cellular extracts)). Increasing the temperature of the micro- and/or nano-emulsion to the subcritical or critical point of the disruption agent is performed by a means (14) for heating comprised in said relief valve (13).

Advantageously, the reduced pressure is comprised between (about) atmospheric pressure and the pressure (in the system) in between the hydrodynamic agitation system (11) and relief valve (13).

According to the present invention, a method is thus provided for disrupting suspended biomass material and (optionally) solubilizing the (valuable) cellular substances (from the disrupted biomass material).

More particularly, it is provided a method for disrupting suspended biomass material, by means of a combination of pressurization and hydrodynamic mixing with, subsequently, a liquid to supercritical to gas phase transition (or phase transition of pressurized liquid disruption agent to supercritical and subsequently to gas phase) for solubilizing (valuable) cellular substances.

More particularly, a method is provided for disrupting suspended biomass material and (optionally) subsequently solubilizing the (valuable) cellular substances (from the disrupted biomass material).

Advantageously, in a method of the invention, valuable cellular extracts (or cellular substances) from substance class of fat, oil, and lipids contained in the biomass material are solubilized.

Alternatively, valuable cellular extracts (or cellular substances) from substance class of carbohydrates contained in the biomass material are solubilized.

Alternatively, valuable cellular extracts (or cellular substances) from substance class of proteins contained in the biomass material are solubilized.

Alternatively, valuable cellular extracts (or cellular substances) from substance class of carotenoids contained in the biomass material are solubilized.

Obtained cellular substances can be used as nutrients, vitamins, for conversion by bacteria in digestions, or in food and pharmaceutical industry (e.g. antibiotics, enzymes, biochemicals).

Preferably, the steps of the method before the step of increasing the temperature to the subcritical or critical point of the disruption agent are performed at a temperature range between (about) 2°C up to (about) 70°C, more preferably between (about) 20°C up to (about) 70°C, even more preferably between (about) 20°C up to (about) 25°C, most preferably at room temperature.

More particularly, the steps of the method of the invention for disrupting the biomass material are performed at a temperature range between (about) 2°C up to (about) 70°C, more preferably between (about) 20°C up to (about) 70°C, even more preferably between (about) 20°C up to (about) 25°C, most preferably at room temperature.

By performing the method of the invention for disrupting biomass material, no extreme temperatures are applied. Therefore, (in addition to the absence of high shear forces acting on the biomass material to be disrupted) a mild (i.e. more gentle) disruption of biomass material is provided (when compared to cell disruption methods described in the art).

Advantageously, in a method of the invention, the disruption agent is recovered (out of or from the third vessel (35)) and returned into the second pump (20) (to be pressurized again). More particularly, the disruption agent is recycled (thereby (further) reducing the total amount of disruption agent used, and reducing the total operational expenditure (or total cost), compared to prior art methods).

Advantageously, the disruption agents used in a method of the invention are known to be used in refrigerant systems and/or in supercritical fluid extractions. Finding a suitable disruption agent for use in (a method of) the invention depends on the product to be obtained from the biomass material.

Advantageously, the disruption agent is selected from the group consisting of ethane, propane, butane, ethylene, propylene, butylene, other saturated or unsaturated hydrocarbons, carbon dioxide, nitrous oxide, dimethyl ether, sulfur hexafluoride, freon, and mixtures thereof.

More advantageously, freon is including chlorofluorocarbons, such as R141 b; or hydrofluorocarbons, such as R134a, R125, or R32.

Alternatively, the disruption agent is not being a hydrocarbon (consisting entirely of hydrogen and carbon atoms).

Alternatively, the disruption agent is a hydrocarbon further comprising one or several heteroatom(s), said heteroatom (preferably) being selected from the group consisting of oxygen, nitrogen, sulfur, and/or one or several halogen(s).

Advantageously, in a method of the invention, a further step of (partially) isolating (or (partially) separating) and selective extracting (the solubilized) cellular valuable substances (from disrupted biomass material) is performed.

More particularly, in a method of the invention, a further step of (partially) isolating (or (partially) separating) and selective extracting (solubilized) proteins (from disrupted biomass material) is performed.

More advantageously, after performing the mild disruption method of the present invention and subsequent solubilization, (the solubilized) proteins are further (partially) isolated (or (partially) separated) and selective extracted from the disrupted biomass material.

More advantageously, the (final) treated suspension (of biomass material, comprising solubilized cellular valuable substances) (passing the relief valve (13) or being present in the third vessel (35)) is (partially) separated by centrifugation and/or filtration, so as to obtain soluble (containing proteins and carbohydrates) and insoluble (being lipid enriched) phases.

In other words, after cell disruption and (optionally) solubilization, supernatant and solid phases are separated by centrifugation and/or filtration.

More advantageously, the (final) treated suspension (passing the relief valve (13) or being present in the third vessel (35)) is separated in a lipid enriched insoluble phase and a soluble phase containing proteins and carbohydrates by centrifugation and/or filtration.

Even more advantageously, the (final) treated suspension is separated (in a lipid enriched insoluble phase and a soluble phase containing proteins and carbohydrates) by centrifugation.

Advantageously, a method of the invention is integrated with other cell disruption methods for increasing the efficiency of the disruption of biomass (however, thereby still assuring an overall mild cell disruption).

Advantageously, the other cell disruption methods are performed on the micro- and/or nano-emulsion coming out of the tubing system (8), but before performing the step of increasing the temperature of the emulsion to the subcritical or critical point of the disruption agent (while transferring it to a third vessel (35) at reduced or atmospheric pressure). Suitable cell disruption methods for integration with a method of the invention will be apparent for those skilled in the art.

Preferably, the method of the invention is integrated with other cell disruption methods selected from a group of mechanical methods consisting of cell disruption by means of ball mill, bead mill, high pressure homogenizer, high speed homogenizer, ultrasonic homogenizer, microwave, ultrasonic bath, french press, pulsed electric fields, pulsed arc, and microfluidizer. More preferably, the method of the invention is integrated with other cell disruption methods selected from a group of mechanical methods consisting of cell disruption by means of high pressure homogenizer, microwave, ultrasonic bath, pulsed electric fields, and microfluidizer.

Alternatively, the method of the invention is integrated with other cell disruption methods selected from a group of chemical methods consisting of cell disruption by means of antibiotics, chelating agents, chaotropes, detergents, hypochlorites, other cosolvents, acid and alkali treatment.

Alternatively, the method of the invention is integrated with other cell disruption methods selected from a group of biological methods consisting of cell disruption by means of enzymes, phages, and autolysis.

Alternatively, the method of the invention can be integrated with other cell disruption methods selected from a group of physical methods consisting of cell disruption by means of freezing and thawing, thermolysis, and explosive decompression.

### EXAMPLES

In the examples described below, the dimensions of the apparatus of the invention used (when including a pressure oscillation system and a sparger system) are the following:
distance from first pump to sparger = 45 cm;
distance from second pump to sparger = 45 cm;
length of pressure oscillation system = 10 cm (i.e. starting from 1^{st} orifice + 3 cm of primary tube + 2^{nd} orifice + 3 cm of primary tube + 3^{rd} orifice + 4 cm of primary tube);
length of hydrodynamic agitation system = 3 subsidiary tubing system of 10*10 cm² each with 2.5 cm primary tube in between them;
distance from hydrodynamic agitation zone to relief valve = 45 cm.

Other suitable dimensions of the apparatus used in a method of the invention will be apparent for those skilled in the art.

### Example 1:

### Solubilization ratio (%) between untreated and treated suspension(s) of biomass

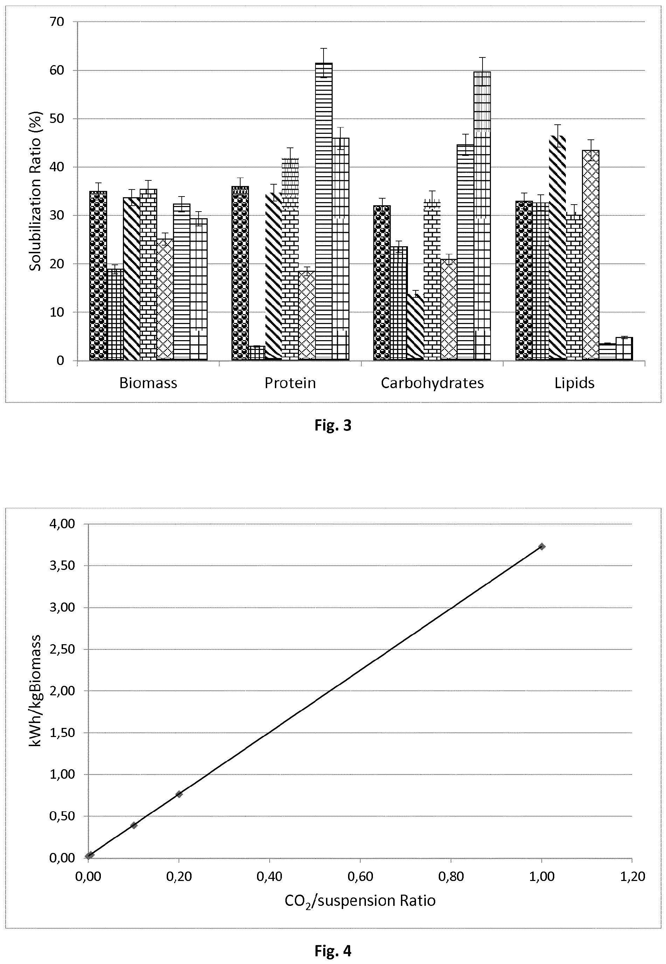

Suspensions of *Neochloris oleoabundans* (unicellular, green microalgae) biomass were treated either by conventional bead milling, conventional explosive decompression method, or the explosive (or rapid) decompression method according to the invention (performed with or without including a pressure oscillation system). The suspension was also treated by a preliminary explosive (or rapid) decompression method of the invention wherein both a sparger system and a pressure oscillation system were not included. After cell disruption and solubilization, the treated suspensions were analyzed to determine the total biochemical compositions, i.e. total lipid, protein, and carbohydrate determinations were carried out. Also the untreated sample of green microalgae biomass was analyzed. The results are summarized in **figure 3****.**

Three runs of **conventional bead milling** were performed on algae suspensions having a dry cell weight of 2.81 % (w/w), 3.59 % (w/w), and 5.84 % (w/w), respectively. The algae suspension was recirculated through the grinding chamber of the agitation bead mill DYNO^{®}-Mill, Type MULTI LAB, (supplied by Willy A. Bachofen AG Maschinenfabrik, Muttenz, Switzerland) for 30 min with a pump speed of 1.5 L/min. The 0.3 L grinding chamber was filled with the maximum amount (approximately 65 % [v/v]) of ceramic beads made of Zirconia, Yttria stabilized, 0.4-0.6 mm. Water, cooled to 5 °C, was continuously circulating in the cooling jacket of the grinding chamber to avoid elevated temperatures inside the chamber. Outside the grinding chamber the algae suspension was kept at 5 °C. Using these process conditions the temperature of the algae suspension at the mill outlet never exceeded 20 °C. The conventional bead milling average results are depicted by 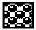 in **figure 3****.**

The **conventional explosive decompression method** is performed inside 100 ml premex high-pressure stirred vessel reactors, at 35°C stable temperature and 10000-20000 kPa (100-200 bar) pressure. Treatment time is 15-45 min, during which 100-300 rpm stirring is performed on a suspension having a (initial) dry cell weight of 6.4 % (w/w). 5 runs are performed to see the effect of the parameters. The best results from conventional explosive decompression runs are depicted by 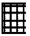 in **figure 3****.**

Performing the **explosive decompression method of the invention without including a pressure oscillation system and without including a sparger system** (also referred to as the **preliminary explosive decompression method of the present invention, without pressure oscillation and without sparger),** the **highest biomass solubilization run** was performed with a CO₂/suspension ratio (w/w) of 241 %, while the relief valve temperature set to 70 °C, the suspension having a dry cell weight of 3.2 % (w/w), the cell suspension flow rate being 4,15 g/min at a pressure of 200 bar, and without any co-solvent addition. 33.7 % of biomass is solubilized. The biochemical composition of the solubilized material was the same (± 3) with untreated biomass. The result from the related run is depicted by 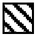 in **figure 3****.**

It is to be noted that in the preliminary explosive decompression method of the invention, the total amount of disruption agent used increases a lot, when compared to the method of the invention performed including a sparger system. In other words, the total amount of disruption agent used is reduced performing the method of the invention including a sparger system, when compared to the preliminary explosive decompression method of the present invention, without pressure oscillation and without sparger.

**Highest biomass and lipid solubilization was achieved in the method of the present invention performed without pressure oscillation** (or in other words, the explosive decompression method of the invention without pressure oscillation). The **highest biomass solubilization run** was performed with a CO₂/suspension ratio (w/w) of 20 %, while the whole system was at room temperature, the suspension having a dry cell weight of 2.6 % (w/w), the cell suspension flow rate being 15 g/min at a pressure of 250 bar, and without any co-solvent addition. 35.41 % of biomass is solubilized. 45.73 % of the solubilized material was protein, while only 35.22 % of the untreated biomass was protein. The best results from the highest biomass solubilization run are depicted by 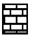 in **figure 3****. The highest lipid solubilization run** was performed with a CO₂/suspension ratio (w/w) of 19.46 %, the hydrodynamic agitation system being cooled down to 4°C, the relief valve being at room temperature, the suspension having a dry cell weight of 2.5 % (w/w), the cell suspension flow rate being 4.8 g/min at a pressure of 75 bars, and with 8 % (w/w) isopropanol addition as co-solvent. 43.43 % of lipids are solubilized. 58.2 % of the solubilized material was lipid. The best results from the highest lipid solubilization run are depicted by 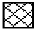 in **figure 3****.**

**Highest protein and carbohydrate solubilization was achieved in the method of the present invention performed with pressure oscillation** (or in other words, the explosive decompression method of the invention with pressure oscillation). **The highest protein solubilization run** was performed with a CO₂/ suspension ratio (w/w) of 25.08 %, while the relief valve is at 45°C, the suspension having a dry cell weight of 1.65 % (w/w), the cell suspension flow rate being 10.5 g/min at a pressure of 175 bar, and without any co-solvent addition. 61.47 % of proteins are solubilized. 77.7 % of the solubilized material were proteins, while only 40.86 % of the untreated biomass were proteins. The best results from the highest protein solubilization run are depicted by 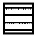 in **figure 3****.** The **highest carbohydrate solubilization run** was performed with a CO₂/ suspension ratio (w/w) of 50.71 %, while the relief valve is at 20°C, the suspension having a dry cell weight of 1.65 % (w/w), the cell suspension flow rate being 15 g/min at a pressure of 225 bar, and without any co-solvent addition. 59.65 % of carbohydrates are solubilized. 41.9 % of the solubilized material were carbohydrates, while only 20.6 % of the untreated biomass were carbohydrates. The best results from the highest carbohydrate solubilization run are depicted by 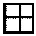 in **figure 3****.**

The data in **figure 3** show that the distribution of biochemicals can be tuned up by changing process parameter values and/or by modifying the design parameters. Using the conventional methods, the system favored lipids, however, the lipid extraction yield was not enough.

In the method of the present invention, cells are enriched at higher liquefied gas pressures (like liquid CO₂) instead of high (but still directly) gas pressure as in prior art conventional explosive decompression methods.

From this example it follows that the present invention thus provides a method (and related apparatus) for tuning the extraction ratios of the cellular substances initially comprised in the biomass material (thereby minimizing the contaminating load).

### Example 2:

### Energy consumption (in kWh/kg of dry biomass) versus CO₂/suspension of biomass material ratio

A suspension of *Neochloris oleoabundans* (unicellular, green microalgae) biomass having 2 % DCW (dry cell weight) concentration is treated using the cell disruption method of the invention.

Bernoulli's equation is used to compute the energy of the fluids before entering the system and when being in the system. The energy, which should be added to the system, to provide a pressure of 250 bar to a 15 g/min single cell suspension and a 0.075-15 g/min CO₂ flow (at room temperature), is calculated by subtracting the total energy in the system and before the system. For the energy calculation, the starting conditions of both the single cell suspension and the CO₂ are assumed being at atmospheric pressure at room temperature.

**Figure 4** represents the energy consumption (in kWh/kg of dry biomass) *versus* the CO₂/suspension ratio, showing that the energy consumption performing the method of the invention is comprised between (about) 0.04 kWh/kgDryBiomass and (about) 3.54 kWh/kgDryBiomass while the ratio of the disruption agent to the suspension of single cell biomass material is ranging between (about) 0.005 to (about) 0.950 kg/kg.

In prior art, to the contrary, much higher amounts of solvent (disruption agent) compared to the amount of biomass (slurry) material are used.

Indeed, for example in US 2011/0183403, it is described that the ratio of solvent (disruption agent) to biogenic suspended raw material (18.1 % DCW) is ranging between 1 kg/kg and 90 kg/kg. The specific examples in said document only mentioning 90 kg/kg and 110 kg/kg (CO₂/suspended biogenic raw material) (with unknown amount at 1500 bar and unknown amount at 300 bar), meaning more energy is consumed when performing the prior art method for disrupting biomass material described in US 2011/0183403, compared to the disruption method of the present invention.

With the method of the invention, the total amount of disruption agent used can thus be reduced and consequently less energy is consumed, thereby reducing the total operational expenditure (or total cost), compared to prior art methods.

From the description and the examples above, it follows that the present invention thus provides an improved method for disrupting suspended biomass material and a related apparatus, which overcome the disadvantages of prior art methods and apparatuses.

In particular, the present invention provides a method and a related apparatus for a physical, non-mechanical, mild disruption of suspended biomass material compared to prior art methods and apparatuses.

Furthermore, the present invention provides such method and related apparatus, obtaining (valuable) cellular substances from suspended biomass material with a high throughput (and low contact time).

The present invention provides a method for disrupting suspended biomass material and related apparatus, (optionally) integrated with a solubilization and/or subsequent selective extraction or separation of the obtained (valuable) cellular substances.

More particularly, it is provided a solubilization of cellular substances from suspended biomass material and minimization of contaminating load by tuning the extraction ratio of the preferred product or biochemical.

Moreover, the present invention provides a method (and related apparatus) for disrupting suspended biomass material with a solubilization and/or subsequent selective extraction or separation of the obtained cellular substances, lowering the total operational expenditure (or total cost) compared to prior art methods and apparatuses and being applicable on industrial scale.

The present invention thus provides a more efficient method compared to prior art methods.

It will be apparent that the stack of (parallel) systems according to the invention (said stack being based on the apparatus of the present invention) overcomes the disadvantages of prior art apparatuses as well.

## Claims

1. A method for disrupting suspended biomass material comprising the steps of:
- providing a suspension of biomass material in a first vessel (1), said suspension having a dry cell weight being comprised between 0.1 % and 15% (w/w);
- pressurizing said suspension to a pressure (p1) being comprised between 7500 kPa and 450000 kPa by using a first high-pressure pump (10);
- providing a pressurized gas disruption agent and/or a pressurized liquid disruption agent in a second vessel (2);
- pressurizing said gas and/or liquid disruption agent to a pressure (p2) being comprised between 7500 kPa and 450000 kPa by using a second high-pressure pump (20), said pressure (p2) being higher than said pressure (p1);
- injecting a stream of said pressurized disruption agent into a stream of said pressurized suspension of biomass material by using a sparger system (7), thereby bringing said two streams together and forming a micro-emulsion;
- passing said micro-emulsion sequentially through a hydrodynamic agitation system (11) and a relief valve (13).

2. The method of claim 1, wherein after the step of using the sparger system (7) and before the step of passing sequentially through the hydrodynamic agitation system (11) and the relief valve (13), said micro-emulsion is passing through a pressure oscillation system (9).

3. The method of claim 1 or 2, wherein simultaneously with the step of passing the micro-emulsion through the relief valve (13), the temperature of the micro-emulsion is increased to the subcritical or critical point of the disruption agent and the process stream coming out of the relief valve (13) is transferred to a third vessel (35) at reduced or atmospheric pressure, thereby solubilizing cellular substances.

4. The method of any one of the preceding claims, wherein the disruption agent is selected from the group consisting of ethane, propane, butane, ethylene, propylene, butylene, other saturated or unsaturated hydrocarbons, carbon dioxide, nitrous oxide, dimethyl ether, sulfur hexafluoride, freon, and mixtures thereof.

5. The method of any one of the preceding claims, wherein the stream of said pressurized disruption agent is injected into the stream of said pressurized suspension through openings (29,30,31,32) of a diameter (d3) comprised between 0.01 mm and 1 mm.

6. The method of any one of the preceding claims, wherein the ratio of the disruption agent to the suspension of biomass material is comprised between 0.001 and 0.95 kg/kg.

7. The method of any one of the preceding claims, wherein the steps of the method before the step of increasing the temperature to the subcritical or critical point of the disruption agent are performed at a temperature range between 2°C up to 70°C.

8. The method of any of claim 3 to 7, wherein the treated suspension of biomass material is separated by centrifugation and/or filtration, so as to obtain soluble and insoluble phases.

9. An apparatus for disrupting suspended biomass material comprising:
at least one first vessel (1) for holding a suspension of biomass material connected to at least one first high-pressure pump (10) by at least one tube (3), said first high-pressure pump (10) being adapted for pressurizing the suspension of biomass material coming from said first vessel (1); at least one second vessel (2) for holding a pressurized gas disruption agent and/or pressurized liquid disruption agent connected to at least one second high-pressure pump (20) by at least one tube (4), said second high-pressure pump (20) being adapted for pressurizing the gas and/or liquid disruption agent coming from said second vessel (2); wherein each of said first and second high-pressure pump (10,20) is connected by at least one tube (5,6) to at least one sparger system (7), said sparger system (7) being adapted for injecting a first stream of pressurized disruption agent into a second stream of a pressurized suspension of biomass material and for forming a micro-emulsion, wherein said sparger system (7) is further connected to a tubing system (8) for mixing said first and second streams, said tubing system (8) comprising a primary tube (15) and at least one hydrodynamic agitation system (11), and said tubing system (8) being further connected to a relief valve (13), and wherein said sparger system (7) comprises a first sparger tube (21) having a diameter (d1) and having two inlets (24,34) and one outlet (28), said first sparger tube (21) comprising a second sparger tube (22) placed along the direction formed by one of the inlets (34) and the outlet (28), said second sparger tube (22) having a diameter (d2) being two times smaller than the diameter (d1) of the first sparger tube, said second sparger tube (22) comprising a distribution of sets (23) of first openings through the surface of the second sparger tube (22) along a width (w), said first openings having a diameter (d3) comprised between 0.01 mm and 1 mm.

10. The apparatus of claim 9, wherein the tubing system (8) comprises at least one pressure oscillation system (9) before the at least one hydrodynamic agitation system (11).

11. The apparatus of claim 10, wherein said pressure oscillation system (9) comprises at least two orifices connected in series, each orifice having a diameter (d8), said diameter (d8) being four to sixty-four times smaller than the diameter (d7) of the primary tube (15).

12. The apparatus of any one of claim 9 to 11, wherein said hydrodynamic agitation system (11) comprises at least one subsidiary tubing system (16) connected by the primary tube (15), wherein the diameter (d9) of the tubes of the subsidiary tubing system (16) is two times smaller, than the diameter (d7) of the primary tube (15).

13. The apparatus of any one of claim 9 to 12, wherein the relief valve (13) comprises a means (14) for heating a stream coming out of the tubing system (8), said relief valve being further connected to a third vessel (35).

14. Use of the apparatus according to any of claim 9 to 13 for disrupting suspended biomass material and, optionally, solubilizing the obtained cellular substances.

15. Use of the apparatus according to claim 14 for further isolating and selective extracting the solubilized cellular substances.

## Patentansprüche

1. Ein Verfahren zum Aufschluss von suspendiertem Biomassematerial, umfassend die folgenden Schritte:
- Bereitstellen einer Suspension von Biomassematerial in einem ersten Behälter (1), wobei die besagte Suspension ein Trockenzellgewicht, das zwischen 0,1 % und 15 % (w/w) liegt, aufweist;
- Beaufschlagen der Suspension mit einem Druck (p1), der zwischen 7.500 kPa und 450.000 kPa liegt, durch Verwenden eine erste Hochdruckpumpe (10);
- Bereitstellen eines druckbeaufschlagten Gasaufschlussmittels und/oder druckbeaufschlagten Flüssigaufschlussmittels in einem zweiten Behälter (2);
- Beaufschlagen des Gas- und/oder Flüssigaufschlussmittels mit einem Druck (p2), der zwischen 7.500 kPa und 450.000 kPa liegt, durch Verwenden einer zweiten Hochdruckpumpe (20), wobei der besagte Druck (p2) höher als der Druck (p1) ist;
- Injizieren eines Stroms des besagten druckbeaufschlagten Aufschlussmittels in einen Strom der besagten druckbeaufschlagen Suspension von Biomassematerial durch Verwenden eines Einblasrohrsystems (7), um dadurch die beiden besagten Ströme zusammenzubringen und eine Mikroemulsion zu bilden;
- sequenzielles Durchlaufen der besagten Mikroemulsion durch ein hydrodynamisches Rührsystem (11) und ein Druckbegrenzungsventil (13).

2. Das Verfahren nach Anspruch 1, wobei nach dem Schritt des Verwendens eines Einblasrohrsystems (7) und vor dem Schritt des sequenziellen Durchlaufens durch das hydrodynamische Rührsystem (11) und das Druckbegrenzungsventil (13), die besagte Mikroemulsion durch ein Druckoszillationssystem (9) durchläuft.

3. Das Verfahren nach Anspruch 1 oder 2, wobei gleichzeitig mit dem Schritt des Durchlaufens der Mikroemulsion durch das Druckbegrenzungsventil (13), die Temperatur der Mikroemulsion auf den subkritischen oder kritischen Punkt des Aufschlussmittels erhöht wird, und der Prozessstrom, der aus dem Druckbegrenzungsventil (13) austritt, bei einem reduzierten oder atmosphärischen Druck zu einem dritten Behälter (35) überführt wird, um dadurch zelluläre Substanzen zu solubilisieren.

4. Das Verfahren nach irgendeinem der vorhergehenden Ansprüche, wobei das Aufschlussmittel aus der Gruppe bestehend aus Ethan, Propan, Butan, Ethylen, Propylen, Butylen, anderen gesättigten oder ungesättigten Kohlenwasserstoffen, Kohlendioxid, Stickstoffoxid, Dimethylether, Schwefelhexafluorid, Freon, und Mischungen davon ausgewählt wird.

5. Das Verfahren nach irgendeinem der vorhergehenden Ansprüche, wobei der Strom des besagten druckbeaufschlagten Aufschlussmittels durch Öffnungen (29, 30, 31, 32) mit einem Durchmesser (d3), der zwischen 0,01 mm und 1 mm liegt, in den Strom der besagten druckbeaufschlagten Suspension injiziert wird.

6. Das Verfahren nach irgendeinem der vorhergehenden Ansprüche, wobei das Verhältnis des Aufschlussmittels zur Suspension von Biomassematerial zwischen 0,001 und 0,95 kg/kg liegt.

7. das Verfahren nach irgendeinem der vorhergehenden Ansprüche, wobei die Schritte des Verfahrens vor dem Schritt des Erhöhens der Temperatur auf den subkritischen oder kritischen Punkt des Aufschlussmittels in einem Temperaturbereich von 2 °C bis 70 °C ausgeführt werden.

8. Das Verfahren nach irgendeinem der Ansprüche 3 bis 7, wobei die behandelte Suspension von Biomassematerial durch Zentrifugieren und/oder Filtrieren getrennt wird, um lösliche und unlösliche Phasen zu erhalten.

9. Eine Vorrichtung zum Aufschließen von suspendiertem Biomassematerial, umfassend:
mindestens einen ersten Behälter (1) zum Halten einer Suspension von Biomassematerial, der durch mindestens ein Rohr (3) mit mindestens einer ersten Hochdruckpumpe (10) verbunden ist, wobei die besagte erste Hochdruckpumpe (10) zum Beaufschlagen der vom besagten ersten Behälter (1) kommenden Suspension von Biomassematerial mit Druck ausgelegt ist;
mindestens einen zweiten Behälter (2) zum Halten eines druckbeaufschlagten Gasaufschlussmittels und/oder druckbeaufschlagten Flüssigaufschlussmittels, der durch mindestens ein Rohr (4) mit mindestens einer zweiten Hochdruckpumpe (20) verbunden ist, wobei die besagte zweite Hochdruckpumpe (20) zum Beaufschlagen der vom zweiten Behälter (2) kommenden Suspension von Biomassematerial mit Druck ausgelegt ist;
wobei jede der ersten und der zweiten Hochdruckpumpe (10, 20) durch mindestens ein Rohr (5, 6) mit mindestens einem Einblasrohrsystem (7) verbunden ist, das besagte Einblasrohrsystem (7) zum Injizieren eines ersten Stroms von druckbeaufschlagtem Aufschlussmittel in einen zweiten Strom einer druckbeaufschlagten Suspension von Biomassematerial und zum Bilden einer Mikroemulsion ausgelegt ist,
wobei das besagte Einblasrohrsystem (7) ferner mit einem Rohrsystem (8) zum Mischen der besagten ersten und zweiten Ströme verbunden ist, das besagte Rohrsystem (8) ein Primärrohr (15) und mindestens ein hydrodynamisches Rührsystem (11) umfasst, und das besagte Rohrsystem (8) ferner mit einem Druckbegrenzungsventil (13) verbunden ist, und
wobei das besagte Einblasrohrsystem (7) ein erstes Injektionsrohr (21) mit einem Durchmesser (d1) und mit zwei Einlässen (24, 34) und einem Auslass (28) umfasst, das erste besagte Injektionsrohr (21) ein zweites Injektionsrohr (22) umfasst, das entlang der Richtung angeordnet ist, die durch einen der Einlässe (34) und den Auslass (28) gebildet wird, das besagte zweite Injektionsrohr (22) einen Durchmesser (d2) aufweist, der zweimal kleiner als der Durchmesser (d1) des ersten Injektionsrohrs ist, das besagte zweite Injektionsrohr (22) eine Verteilung von Sätzen (23) von ersten Öffnungen durch die Oberfläche des zweiten Injektionsrohrs (22) entlang einer Breite (w) umfasst, wobei die ersten Öffnungen einen Durchmesser (d3) aufweisen, der zwischen 0,01 mm und 1 mm liegt.

10. Die Vorrichtung nach Anspruch 9, wobei das Rohrsystem (8) mindestens ein Druckoszillationssystem (9) vor dem mindestens einen hydrodynamischen Rührsystem (11) umfasst.

11. Die Vorrichtung nach Anspruch 10, wobei das Druckoszillationssystem (9) mindestens zwei in Reihe verbundene Bohrungen umfasst, wobei jede Bohrung einen Durchmesser (d8) aufweist, wobei der Durchmesser (d8) vier bis vierundsechzigmal kleiner als der Durchmesser (d7) des Primärrohrs (15) ist.

12. Die Vorrichtung nach irgendeinem der Ansprüche 9 bis 11, wobei das besagte hydrodynamische Rührsystem (11) mindestens ein ergänzendes Rohrsystem (16) umfasst, das durch das Primärrohr (15) verbunden ist, wobei der Durchmesser (d9) der Rohre des ergänzenden Rohrsystems (16) zweimal kleiner als der Durchmesser (d7) des Primärrohrs (15) ist.

13. Die Vorrichtung nach irgendeinem der Ansprüche 9 bis 12, wobei das Druckbegrenzungsventil (13) ein Mittel (14) zum Erwärmen eines Stroms, der aus dem Rohrsystem (8) kommt, umfasst, wobei das besagte Druckbegrenzungsventil ferner mit einem dritten Behälter (35) verbunden ist.

14. Verwendung der vorrichtung nach irgendeinem der Ansprüche 9 bis 13 zum Aufschließen von suspendiertem Biomassematerial und, optional, Solubilisieren der erhaltenen Zellsubstanzen.

15. Verwendung der Vorrichtung nach Anspruch 14 zum weiteren Isolieren und selektiven Extrahieren von solubilisierten Zellsubstanzen.

## Revendications

1. Procédé pour dissocier une matière de biomasse en suspension, comprenant les étapes consistant à :
prévoir une suspension de matière de biomasse dans une première cuve (1), ladite suspension ayant un poids cellulaire sec compris entre 0,1% et 15% (w/w) ;
mettre sous pression ladite suspension à une pression (p1) qui est comprise entre 7500 kPa et 450000 kPa en utilisant une première pompe haute pression (10) ;
prévoir un agent de dissociation de gaz sous pression et/ou un agent de dissociation de liquide sous pression dans une deuxième cuve (2) ;
mettre sous pression ledit agent de dissociation de gaz et/ou de liquide à une pression (p2) qui est comprise entre 7500 kPa et 450000 kPa en utilisant une seconde pompe haute pression (20), ladite pression (p2) étant supérieure à ladite pression (p1) ;
injecter un flux d'agent de dissociation sous pression dans un flux de ladite suspension de matière de biomasse sous pression en utilisant un système disperseur (7), rassemblant ainsi lesdits deux flux et formant une microémulsion ;
faire passer ladite microémulsion de manière séquentielle par un système d'agitation hydrodynamique (11) et une valve de décharge (13).

2. Procédé selon la revendication 1, dans lequel après l'étape consistant à utiliser le système disperseur (7) et avant l'étape consistant à passer de manière séquentielle par le système d'agitation hydrodynamique (11) et la valve de décharge (13), ladite microémulsion passe par un système d'oscillation de pression (9).

3. Procédé selon la revendication 1 ou 2, dans lequel simultanément avec l'étape consistant à faire passer la microémulsion par la valve de décharge (13), la température de la microémulsion est augmentée jusqu'à un point sous-critique ou critique de l'agent de dissociation et le flux de traitement sortant de la valve de décharge (13) est transféré à une troisième cuve (35) à une pression réduite ou atmosphérique, solubilisant ainsi les substances cellulaires.

4. Procédé selon l'une quelconque des revendications précédentes, dans lequel l'agent de dissociation est choisi dans le groupe comprenant l'éthane, le propane, le butane, l'éthylène, le propylène, le butylène, d'autres hydrocarbures saturés ou insaturés, le dioxyde de carbone, l'oxyde azoté, l'éther diméthylique, l'hexafluorure de soufre, le fréon et leurs mélanges.

5. Procédé selon l'une quelconque des revendications précédentes, dans lequel le flux dudit agent de dissociation sous pression est injecté dans le flux de ladite suspension sous pression par des ouvertures (29, 30, 31, 32) d'un diamètre (d3) compris entre 0,01 mm et 1 mm.

6. Procédé selon l'une quelconque des revendications précédentes, dans lequel le rapport de l'agent de dissociation sur la suspension de matière de biomasse est compris entre 0,001 et 0,95 kg/kg.

7. Procédé selon l'une quelconque des revendications précédentes, dans lequel les étapes du procédé avant l'étape consistant à augmenter la température jusqu'au point sous-critique ou critique de l'agent de dissociation sont réalisées dans une plage de température comprise entre 2°C à 70°C.

8. Procédé selon l'une quelconque des revendications 3 à 7, dans lequel la suspension traitée de matière de biomasse est séparée par centrifugation et/ou filtration, afin d'obtenir des phases solubles et insolubles.

9. Appareil pour dissocier une matière de biomasse en suspension comprenant :
au moins une première cuve (1) pour contenir une suspension de matière de biomasse raccordée à au moins une première pompe haute pression (10) par au moins un tube (3), ladite première pompe haute pression (10) étant adaptée pour mettre sous pression la suspension de matière de biomasse venant de ladite première cuve (1) ; et moins une deuxième cuve (2) pour contenir un agent de dissociation de gaz sous pression et/ou un agent de dissociation de liquide sous pression, raccordée à au moins une seconde pompe haute pression (20) par au moins un tube (4), ladite seconde pompe haute pression (20) étant adaptée pour mettre sous pression l'agent de dissociation de gaz et/ou de liquide provenant de ladite deuxième cuve (2) ; dans lequel chacune desdites première et seconde pompes haute pression (10, 20) est raccordée par au moins un tube (5, 6) à au moins un système disperseur (7), ledit système disperseur (7) étant adapté pour injecter un premier flux d'agent de dissociation sous pression dans un second flux de suspension de matière de biomasse sous pression et pour former une microémulsion, dans lequel ledit système disperseur (7) est en outre raccordé à un système de tubes (8) pour mélanger lesdits premier et second flux, ledit système de tubes (8) comprenant un tube primaire (15) et au moins un système d'agitation hydrodynamique (11) et ledit système de tubes (8) étant en outre raccordé à une valve de décharge (13), et dans lequel ledit système disperseur (7) comprend un premier tube disperseur (21) ayant un diamètre (d1) et ayant deux entrées (24, 34) et une sortie (28), ledit premier tube disperseur (21) comprenant un second tube disperseur (22) placé le long de la direction formée par l'une parmi les entrées (34) et la sortie (28), ledit second tube disperseur (22) ayant un diamètre (d2) qui est deux fois plus petit que le diamètre (d1) du premier tube disperseur, ledit second tube disperseur (22) comprenant une répartition d'ensembles (23) de premières ouvertures à travers la surface du second tube disperseur (22) le long d'une largeur (w), lesdites premières ouvertures ayant un diamètre (d3) compris entre 0,01 mm et 1 mm.

10. Appareil selon la revendication 9, dans lequel le système de tubes (8) comprend au moins un système d'oscillation de pression (9) avant le au moins un système d'agitation hydrodynamique (11).

11. Appareil selon la revendication 10, dans lequel ledit système d'oscillation de pression (9) comprend au moins deux orifices raccordés en série, chaque orifice ayant un diamètre (d8), ledit diamètre (d8) étant de quatre à soixante-quatre fois plus petit que le diamètre (d7) du tube primaire (15).

12. Appareil selon l'une quelconque des revendications 9 à 11, dans lequel ledit système d'agitation hydrodynamique (11) comprend au moins un système de tubes secondaire (16) raccordé par le tube primaire (15), dans lequel le diamètre (d9) des tubes du système de tubes secondaire (16) est deux fois plus petit que le diamètre (d7) du tube primaire (15).

13. Appareil selon l'une quelconque des revendications 9 à 12, dans lequel la valve de décharge (13) comprend un moyen (14) pour chauffer un flux sortant du système de tubes (8), ladite valve de décharge étant en outre raccordée à une troisième cuve (35).

14. Utilisation de l'appareil selon l'une quelconque des revendications 9 à 13 pour dissocier une matière de biomasse en suspension et, facultativement, pour solubiliser les substances cellulaires obtenues.

15. Utilisation de l'appareil selon la revendication 14 pour de plus isoler et extraire de manière sélective les substances cellulaires solubilisées.
